# EUROPEAN PATENT SPECIFICATION

(11) **EP 3 187 874 B1**
(45) Date of publication and mention of the grant of the patent: **30.09.2020**
(21) Application number: 15836585.8
(22) Date of filing: 14.07.2015
(51) Int. Cl.: G01N 33/543, G01N 21/41, G01N 21/64, G01N 33/553, G01N 21/05, G01N 21/552, G01N 21/03

(54) **DETECTION METHOD AND DETECTION DEVICE**
DETEKTIONSVERFAHREN UND DETEKTIONSVORRICHTUNG
PROCÉDÉ DE DÉTECTION ET DISPOSITIF DE DÉTECTION

(30) Priority: 25.08.2014 JP 2014170438
(43) Date of publication of application: 05.07.2017
(73) Proprietor: Konica Minolta, Inc., Tokyo 100-7015 (JP)
(72) Inventor: MURAYAMA, Takanori, Tokyo 100-7015 (JP)
(74) Representative: Burton, Nick
(86) International application number: PCT/JP2015/070150
(87) International publication number: WO 2016/031412

(56) References cited:
- WO-A1-2007/145180
- WO-A1-2011/074373
- WO-A1-2013/002193
- WO-A1-2013/038914
- WO-A1-2014/087802
- WO-A1-2014/097877
- WO-A2-2013/106458
- JP-A- 2006 292 472
- JP-A- 2006 322 854
- JP-A- 2007 147 420
- JP-A- 2010 112 748
- JP-A- 2012 163 470
- US-A1- 2005 255 000
- US-A1- 2006 078 985
- US-A1- 2014 170 772

## Description

### Technical Field

The present invention relates to a detection method and a detection device for detecting a detection target substance using a detection chip.

### Background Art

In a clinical test or the like, if a small amount of detection target substance such as a protein or DNA in a specimen can be detected quantitatively with a high sensitivity, a treatment can be performed by recognizing a patient's condition rapidly. Therefore, a method capable of detecting a detection target substance in a specimen quantitatively with a high sensitivity has been desired.

In recent years, in order to analyze a small amount of detection target substance, a micro total analysis system (µ-TAS) has been used. In the micro total analysis system, analysis can be performed using small amounts of reagents and samples in a short time advantageously. Examples of the micro total analysis system include a microchannel system using a microchannel chip having a fine channel. A reaction field to which a capturing body for capturing a detection target substance is fixed is disposed in the channel of the microchannel chip. In this reaction field, an antigen-antibody reaction to bond a detection target substance included in a specimen supplied into the channel to the capturing body (hereinafter, also referred to as "primary reaction") and the like are performed. This microchannel system detects a detection target substance captured by the capturing body.

However, the detection target substance can adhere also to an inner surface of the channel nonspecifically. The detection target substance adhering to the inner surface of the channel outside the reaction field is not detected. Therefore, a reliable detection result cannot be obtained disadvantageously. Therefore, as a means for preventing nonspecific adhesion of the detection target substance to the inner surface of the channel, it has been proposed to subject the inner surface of the channel to a blocking treatment (for example, refer to JP 2006-292472).

In a micro total analysis system described in JP 2006-292472, the blocking treatment is performed as a separate step from the primary reaction. Specifically, the inner surface of the channel is subjected to the blocking treatment. Thereafter, a blocking liquid is removed from the inside of the channel, and the inside of the channel is cleaned. Thereafter, a specimen including a detection target substance is introduced into the channel, and the primary reaction is performed in a reaction field. This prevents nonspecific adhesion of the detection target substance to the inner surface of the channel in the micro total analysis system described in JP 2006-292472.

WO 2013/038914 describes a method and device for quantitative surface plasmon-field enhanced fluorescence spectroscopy. After SPR measurement has been used to measure a total analyte concentration, a fluorescently labeled probe is used to label a specific analyte, and SPFS measurement is used to measure the concentration of said analyte. The ratio of the amount of the specific analyte to the total analyte amount is computed from the total analyte concentration and the concentration of the specific analyte. A detection chip includes a channel with a flow path including a ligand that specifically binds with a specific analyte. A primary antibody is circulated on a SAM film within the channel to produce the capturing body and then a non-specific adsorption prevention treatment is applied in the flow path by circulating and feeding phosphate buffered saline (PBS) containing bovine serum albumin (BSA). A standard antigen solution is then applied to the detection chip. The solutions may be circulated in various ways including feeding liquid in one direction, and reciprocating liquid transfer in both directions.

JP 2006-322854 describes a measuring instrument utilizing total reflection damping capable of adjusting the thrust quantity of a sensor unit so as to optimize it. A Z-direction pressing mechanism includes a stepping motor, a slider, a connecting member, a pressing member and a digital dial gauge. The pressing member is moved in a Z-direction to press a sensor unit against a rail. The position in the height direction of the pressing member is measured by the digital dial gauge and the sensor unit is crushed by the pressing member to be elastically deformed. The pressing member is allowed to fall by predetermined quantity, while confirming the measured value of the digital dial gauge to acquire the Surface Plasmon Resonance (SPR) signal for each elastic deformation quantity of the sensor unit. The elastic deformation quantity of the sensor unit that serves as the optimum pressing quantity is determined, on the basis of the SPR signal acquired.

JP 2010-112748 describes a SPFS detection method for detecting the amount of a substance to be detected. A sample is supplied to the sensor part on a sensor chip 10 and irradiated with exciting light to produce an intensified photoelectric field on the sensor part. A fluorescent label is excited and the amount of the substance to be detected is detected on the basis of the quantity of the light caused by this excitation. A blocking substance, of which the properties are similar to those of the fluorescent substance, for blocking the surface of the sensor part and the fluorescent substance obtained by including a plurality of fluorescent coloring matter molecules by a light transmitting material, which permits transmission of the fluorescence produced from a plurality of the fluorescent coloring matter molecules, are used. Then, the non-specific adsorption of the fluorescent substance onto the sensor part is prevented by the blocking substance. The blocking solution, labelling solution and sample solution may be flowed through the sensor chip together.

When the inside of the channel is cleaned after the blocking treatment, detection time is increased due to increase in the number of steps. In addition, a blocking agent may be peeled off from the inner surface of the channel by cleaning, and an effect of the blocking treatment may be reduced. That is, there is room for improvement of the micro total analysis system described in JP 2006-292472 from viewpoints of reducing detection time and improving a detection accuracy.

WO 2013/038914 A1 discloses a measurement apparatus and method for measurement of a specific analyte. The measurement apparatus comprises a prism-shaped dielectric member and a metal film formed on a horizontal upper surface of the dielectric member. Incident light from a light source is reflected on the outer side of the dielectric member to a light receiving means. The sensor chip, the light source and the light receiving means constitute an SPR measuring section which performs the SPR measurement of the quantitative measurement apparatus. A flow path is formed on the upper surface of the metal film, this surface comprises a ligand that specifically binds with a specific analyte which binds to a fluorescently labelled probe. Sample liquid containing a specific analyte is provided to the flow path and the analyte is coupled to the sensor unit.

A first object of the present invention is to provide a detection method and a detection device capable of detecting a detection target substance in a short time with a high accuracy without reducing an effect of a blocking treatment to an inner surface of a channel by using a reaction method.

A first aspect of the invention provides a detection method according to claim 1.

A second aspect of the invention provides a detection device according to claim 3.

A detection method and a detection device according to the present invention make it possible to detect presence or the amount of a detection target substance in a short time with a high accuracy by using a reaction method that makes it possible to bond a capturing body to a reaction target substance (detection target substance) efficiently in a short time without reducing an effect of a blocking treatment to an inner surface of a channel.

### Brief Description of Drawings

Fig. 1 is a schematic diagram illustrating a configuration of a surface plasmon-field enhanced fluorescence spectroscopy device (SPFS device) according to an embodiment.
Fig. 2 is a flowchart illustrating a first operation procedure of the SPFS device illustrated in Fig. 1.
Fig. 3 is a flowchart illustrating a second operation procedure of the SPFS device illustrated in Fig. 1.
Fig. 4A is a graph indicating a relationship between a flow rate of a blocking liquid and a blocking ratio when a primary reaction is performed following a blocking treatment, and Fig. 4B is a graph indicating a relationship between a flow rate of a mixed liquid and a blocking ratio when the blocking treatment and the primary reaction are performed simultaneously.
Fig. 5A is a graph indicating a relationship between presence or absence of movement of a blocking liquid and the concentration of a blocking agent, and a signal value indicating the amount of a detection target substance when a primary reaction is performed following a blocking treatment, and Fig. 5B is a graph indicating a relationship between the concentration of a blocking agent and a signal value indicating the amount of a detection target substance when the blocking treatment and the primary reaction are performed simultaneously.

### Description of Embodiments

Hereinafter, an embodiment of the present invention will be described in detail with reference to the drawings. Here, as a typical example of a detection device according to the present invention, an analyzing device (SPFS device) utilizing a surface plasmon-field enhanced fluorescence spectroscopy (hereinafter, abbreviated as "SPFS") for detecting the amount of a detection target substance (reaction target substance) in a specimen (liquid containing a reaction target substance) will be described.

### (Configurations of SPFS device and detection chip)

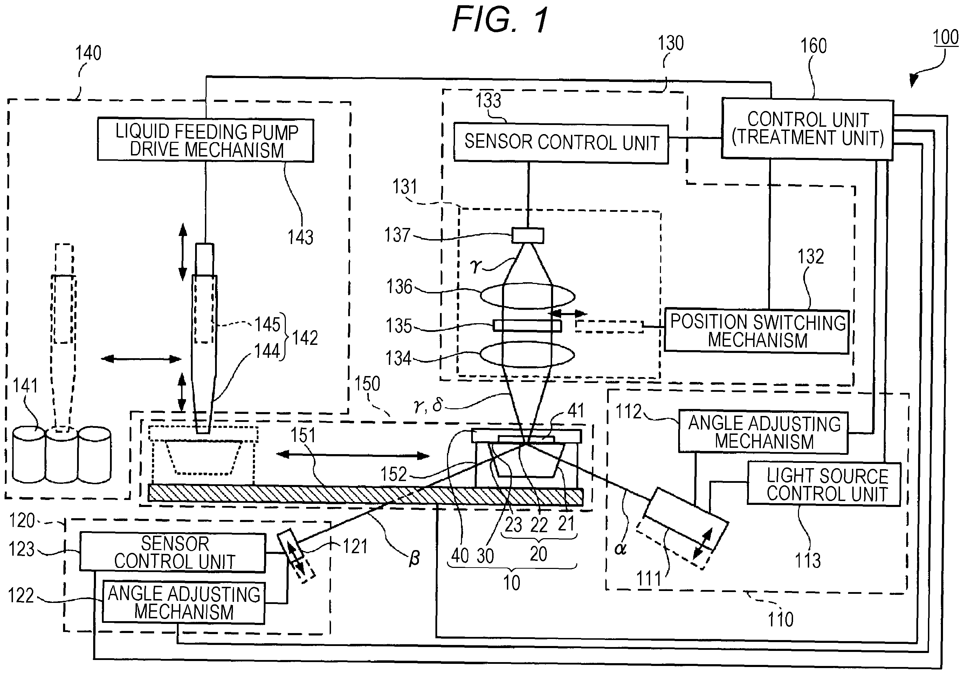

Fig. 1 is a schematic diagram illustrating a configuration of a surface plasmon-field enhanced fluorescence spectroscopy device (SPFS device) 100 according to an embodiment of the present invention. As illustrated in Fig. 1, the SPFS device 100 includes an excitation light irradiation unit (light irradiation unit) 110, a reflected light detecting unit 120, a fluorescence detecting unit 130, a liquid feeding unit 140, a conveying unit 150, and a control unit 160. The SPFS device 100 is used while a detection chip 10 (container) is mounted in a chip holder 152 of the conveying unit 150. Therefore, the detection chip 10 will be described first, and then components of the SPFS device 100 will be described.

The detection chip 10 includes a prism 20 having an incident surface 21, a film forming surface 22, and a light emitting surface 23, a metal film 30 formed on the film forming surface 22, and a channel lid 40 disposed on the metal film 30. As described below, the detection chip 10 (container) includes a channel 41 (containing unit) and a reaction field exposed to the channel 41. In the reaction field, capture of a detection target substance (reaction target substance) by a capturing body, labeling of the detection target substance by a fluorescence substance, or the like is performed. The detection chip 10 is usually replaced whenever analysis is performed. The detection chip 10 is a structure preferably having each length of several mm to several cm, but may be a smaller or larger structure not included in a category of the "chip".

The prism 20 is formed of a dielectric transparent with respect to excitation light α. The prism 20 includes the incident surface 21, the film forming surface 22, and the light emitting surface 23. The incident surface 21 allows the excitation light α from the excitation light irradiation unit 110 to enter the prism 20. The metal film 30 is disposed on the film forming surface 22. The excitation light α which has entered the prism 20 is reflected on a back surface of the metal film 30 to become reflected light β. More specifically, the excitation light α is reflected on an interface (film forming surface 22) between the prism 20 and the metal film 30 to become the reflected light β. The light emitting surface 23 emits the reflected light β outside the prism 20.

The shape of the prism 20 is not particularly limited. In the present embodiment, the shape of the prism 20 is a columnar body having a trapezoidal bottom surface. A surface corresponding to one of bases of the trapezoid is the film forming surface 22, a surface corresponding to one of legs is the incident surface 21, and a surface corresponding to the other leg is the light emitting surface 23. The trapezoid as the bottom surface is preferably an isosceles trapezoid. This makes the incident surface 21 and the light emitting surface 23 symmetric to each other, and makes it difficult for an S-wave component of the excitation light α to stay in the prism 20.

The incident surface 21 is formed such that the excitation light α does not return to the excitation light irradiation unit 110. When a light source of the excitation light α is a laser diode (hereinafter, also referred to as "LD"), return of the excitation light α to the LD disturbs an excitation state of the LD, and causes a wavelength of the excitation light α or an output thereof to fluctuate. Therefore, an angle of the incident surface 21 is set such that the excitation light α is not vertically incident on the incident surface 21 in a scanning range around an ideal resonance angle or enhancement angle. Here, the "resonance angle" means an incident angle when a light quantity of the reflected light β emitted from the light emitting surface 23 is minimized in scanning for an incident angle of the excitation light α with respect to the metal film 30. The "enhancement angle" means an incident angle when a light quantity of scattered light having the same wavelength as the excitation light α emitted above the detection chip 10 (hereinafter, referred to as "plasmon scattered light") δ is maximized in scanning for an incident angle of the excitation light α with respect to the metal film 30. In the present embodiment, each of an angle between the incident surface 21 and the film forming surface 22 and an angle between the film forming surface 22 and the light emitting surface 23 is about 80°.

Design of the detection chip 10 generally determines the resonance angle (and the enhancement angle extremely close thereto) . Examples of a design element include a refractive index of the prism 20, a refractive index of the metal film 30, a film thickness of the metal film 30, an extinction coefficient of the metal film 30, and a wavelength of the excitation light α. The resonance angle and the enhanced angle are shifted by a detection target substance captured on the metal film 30, but the amount thereof is less than a few degrees.

The prism 20 has not a little birefringence characteristic. Examples of a material of the prism 20 include resin and glass. A material of the prism 20 is preferably a resin having a refractive index of 1.4 to 1.6 and a small birefringence.

The metal film 30 is disposed on the film forming surface 22 of the prism 20. This can cause surface plasmon resonance (hereinafter, abbreviated as "SPR") between a photon of the excitation light α incident on the film forming surface 22 under total reflection conditions and a free electron in the metal film 30, and can cause localized field light (generally, also referred to as "evanescent light" or "near-field light") on a surface of the metal film 30. In the present embodiment, the metal film 30 is formed on the entire surface of the film forming surface 22.

A material of the metal film 30 is not particularly limited as long as being a metal capable of causing surface plasmon resonance. Examples of a material of the metal film 30 include gold, silver, copper, aluminum, and an alloy thereof. In the present embodiment, the metal film 30 is a gold thin film. A method for forming the metal film 30 is not particularly limited. Examples of a method for forming the metal film 30 include sputtering, vapor deposition, and plating. The thickness of the metal film 30 is not particularly limited, but is preferably from 30 to 70 nm.

A capturing body for capturing a detection target substance is fixed to a surface of the metal film 30 not facing the prism 20 (surface of the metal film 30), although not particularly illustrated. By fixing the capturing body, a detection target substance can be detected selectively. In the present embodiment, the capturing body is uniformly fixed in a predetermined region (reaction field) on the metal film 30. The kind of the capturing body is not particularly limited as long as the capturing body can capture a detection target substance. In the present embodiment, the capturing body is an antibody specifically bonded to a detection target substance or a fragment thereof. The capturing body is usually stored with a protective layer when the capturing body is not in use from a viewpoint of preventing denaturation caused by drying.

The channel lid 40 is disposed on the metal film 30. When the metal film 30 is formed only on a part of the film forming surface 22 of the prism 20, the channel lid 40 may be disposed on the film forming surface 22. In the present embodiment, the channel lid 40 is disposed on the metal film 30. A channel groove is formed on a back surface of the channel lid 40. The channel lid 40 forms a containing unit for containing a liquid together with the metal film 30 (and the prism 20). In the present embodiment, the containing unit is the channel 41 in which a liquid flows . A moving rate of a liquid in the channel 41 can be adjusted by a size of a cross-sectional area of the channel 41. A cross-sectional area of the channel 41 in a cross section perpendicular to a flowing direction of a liquid on a reaction field described below is not particularly limited, but for example, is preferably 0.01 mm² or more and 100 mm² or less. The cross-sectional area is more preferably 0.01 mm² or more and 10 mm² or less, and still more preferably 0.01 mm² or more and 1 mm² or less from a viewpoint of moving a liquid at a higher speed in the channel 41. The reaction field is disposed so as to be exposed to the channel 41 (containing unit). Examples of the liquid include a specimen containing a detection target substance (for example, blood, serum, plasma, urine, a nasal fluid, saliva, or semen), a blocking liquid containing a blocking agent, a labeling liquid containing a capturing body labeled with a fluorescent substance, a standard liquid, and a cleaning liquid. The capturing body fixed to the metal film 30 is exposed to the inside of the channel 41. Both ends of the channel 41 are connected to an inlet and an outlet (not illustrated) formed on an upper surface of the channel lid 40. When a liquid is supplied into the channel 41, the liquid comes into contact with the capturing body.

The channel lid 40 is preferably formed of a material transparent with respect to fluorescence γ and the plasmon scattered light δ emitted from an upper surface of the metal film 30. Examples of a material of the channel lid 40 include resin. When a portion to extract the fluorescent γ and the plasmon scattered light δ to an outside is transparent with respect to the fluorescence γ and the plasmon scattered light δ, the other portions of the channel lid 40 may be formed of an opaque material. For example, the channel lid 40 is joined to the metal film 30 or the prism 20 by adhesion using a double-sided tape or an adhesive, laser welding, ultrasonic welding, or crimping using a clamp member.

The detection chip 10 may include a frame body having a through hole and a top plate stacked on the frame body in place of the channel lid 40. In this case, the channel 41 (containing unit) is formed by stacking the frame body and the top plate in this order on the prism 20 having the metal film 30 formed. A surface of the metal film 30 becomes a bottom surface of the channel 41. An inner surface of the through hole of the frame body becomes a side surface of the channel 41. One surface (inner surface) of the top plate becomes a top surface of the channel 41.

As illustrated in Fig. 1, the excitation light α enters the prism 20 from the incident surface 21. The excitation light α which has entered the prism 20 is incident on the metal film 30 at a total reflection angle (angle at which SPR occurs). By irradiating the metal film 30 with the excitation light α at an angle at which SPR occurs, localized field light can be generated on the metal film 30. This localized field light excites a fluorescence substance to label a detection target substance present on the metal film 30, and the fluorescence γ is emitted from the vicinity of a surface of the metal film 30 on a side of the channel 41 (containing unit). The SPFS device 100 detects presence or the amount of the detection target substance by measuring a light quantity of the fluorescence γ emitted from the fluorescence substance.

Next, components of the SPFS device 100 will be described. As described above, the SPFS device 100 includes the excitation light irradiation unit 110, the reflected light detecting unit 120, the fluorescence detecting unit 130, the liquid feeding unit 140, the conveying unit 150, and the control unit 160.

The excitation light irradiation unit 110 irradiates the detection chip 10 held by the chip holder 152 with the excitation light α. When the fluorescence γ or the plasmon scattered light δ is measured, the excitation light irradiation unit 110 emits only P-wave with respect to the metal film 30 toward the incident surface 21 such that an incident angle thereof with respect to the metal film 30 becomes an angle to cause SPR. Here, the "excitation light" is light to excite a fluorescence substance directly or indirectly. For example, the excitation light α is light to generate localized field light to excite a fluorescence substance on a surface of the metal film 30 when the metal film 30 is irradiated with the excitation light α through the prism 20 at an angle at which SPR occurs. The excitation light irradiation unit 110 includes a light source unit 111, an angle adjusting mechanism 112, and a light source control unit 113.

The light source unit 111 emits the collimated excitation light α having a constant wavelength and light quantity such that an irradiation spot on a back surface of the metal film 30 has a substantially circular shape. For example, the light source unit 111 includes a light source of the excitation light α, a beam shaping optical system, an APC mechanism, and a temperature adjusting mechanism (none of them is illustrated).

The kind of a light source is not particularly limited, but is a laser diode (LD), for example. Examples of a light source further include a light emitting diode, a mercury lamp, and other laser light sources. When light emitted from a light source is not a beam, the light emitted from the light source is converted into a beam by a lens, a mirror, a slit, or the like. When light emitted from a light source is not monochromatic light, the light emitted from the light source is converted into monochromatic light by a diffraction grating or the like. When light emitted from a light source is not linearly polarized light, the light emitted from the light source is converted into linearly polarized light by a polarizer or the like.

For example, the beam shaping optical system includes a collimator, a bandpass filter, a linearly polarizing filter, a half-wave plate, a slit, and a zooming means. The beam shaping optical system may include all of these means, or may include a part thereof. The collimator collimates the excitation light α emitted from a light source. The bandpass filter changes the excitation light α emitted from a light source into narrow band light having only a center wavelength. This is because the excitation light α from a light source has a small wavelength distribution width. The linearly polarizing filter changes the excitation light α emitted from a light source into completely linearly polarized light. The half-wave plate adjusts a polarization direction of the excitation light α such that a P-wave component is incident on the metal film 30. The slit and zooming means adjust a beam diameter of the excitation light α and a contour shape thereof such that the irradiation spot on the back surface of the metal film 30 has a circular shape having a predetermined size.

The APC mechanism controls a light source such that an output of the light source is constant. More specifically, the APC mechanism detects a light quantity of light branching from the excitation light α with a photodiode (not illustrated) or the like. The APC mechanism controls an output of a light source constantly by controlling input energy in a regression circuit.

Examples of the temperature adjusting mechanism include a heater and a Peltier element. A wavelength and energy of light emitted from a light source may vary according to a temperature. Therefore, by keeping the temperature of a light source constantly by the temperature adjusting mechanism, the wavelength and energy of light emitted from a light source is controlled constantly.

The angle adjusting mechanism 112 adjusts an incident angle of the excitation light α with respect to the metal film 30 (an interface (film forming surface 22) between the prism 20 and the metal film 30). The angle adjusting mechanism 112 rotates an optical axis of the excitation light α and the chip holder 152 relatively in order to emit the excitation light α toward a predetermined position of the metal film 30 through the prism 20 at a predetermined incident angle.

For example, the angle adjusting mechanism 112 rotates the light source unit 111 around an axis (axis perpendicular to paper face of Fig. 1) perpendicular to the optical axis of the excitation light α. At this time, a position of the rotational axis is set such that a position of an irradiation spot on the metal film 30 is hardly changed even when scanning for an incident angle is performed. Particularly by setting the position of the rotation center to the vicinity of an intersection of the two optical axes of the excitation light α at both ends of a scanning range for an incident angle (between the irradiation position on the film forming surface 22 and the incident surface 21), deviation of the irradiation position can be minimized.

As described above, among incident angles of the excitation light α with respect to the metal film 30, an angle at which a light amount of the plasmon scattered light δ is maximum is an enhanced angle. By setting an incident angle of the excitation light α to the enhanced angle or an angle in the vicinity thereof, the fluorescence γ having a high intensity can be measured. Basic incident conditions of the excitation light α are determined by a material of the prism 20 of the detection chip 10 and a shape thereof, a film thickness of the metal film 30, a refractive index of a liquid in the channel 41, and the like. However, an optimum incident condition is varied slightly according to the kind of the fluorescence substance in the channel 41, the amount thereof, a shape error of the prism 20, and the like. Therefore, an optimum enhancement angle is preferably determined for each measurement.

The light source control unit 113 controls various devices included in the light source unit 111 to control emission of the excitation light α from the light source unit 111. For example, the light source control unit 113 is constituted by a known computer or microcomputer including a computing device, a control device, a storage device, an input device, and an output device.

The reflected light detecting unit 120 measures a light quantity of the reflected light β generated by irradiation to the detection chip 10 with the excitation light α for measurement of a resonance angle or the like. The reflected light detecting unit 120 includes a light receiving sensor 121, an angle adjustment mechanism 122, and a sensor control unit 123.

The light receiving sensor 121 is disposed at a position on which the reflected light β is incident, and measures a light quantity of the reflected light β. The kind of the light receiving sensor 121 is not particularly limited. For example, the light receiving sensor 121 is a photodiode (PD) .

The angle adjustment mechanism 122 adjusts a position (angle) of the light receiving sensor 121 according to an incident angle of the excitation light α with respect to the metal film 30. The angle adjustment mechanism 122 rotates the light receiving sensor 121 and the chip holder 152 relatively such that the reflected light β is incident on the light receiving sensor 121.

The sensor control unit 123 controls detection of an output value of the light receiving sensor 121, management of a sensitivity of the light receiving sensor 121 with the detected output value, change of the sensitivity of the light receiving sensor 121 for obtaining a proper output value, and the like. For example, the sensor control unit 123 is constituted by a known computer or microcomputer including a computing device, a control device, a storage device, an input device, and an output device.

The fluorescence detecting unit 130 detects the fluorescence γ generated by irradiation to the metal film 30 with the excitation light α. In addition, the fluorescence detecting unit 130 detects the plasmon scattered light δ generated by irradiation to the metal film 30 with the excitation light α, if necessary. The fluorescence detecting unit 130 includes a light receiving unit 131, a position switching mechanism 132, and a sensor control unit 133.

The light receiving unit 131 is disposed in a normal direction of the metal film 30 of the detection chip 10. The light receiving unit 131 includes a first lens 134, an optical filter 135, a second lens 136, and a light receiving sensor 137.

For example, the first lens 134 is a condenser lens, and condenses light emitted from an upper surface of the metal film 30. For example, the second lens 136 is an imaging lens, and images the light condensed by the first lens 134 on a light receiving surface of the light receiving sensor 137. An optical path between the two lenses is substantially parallel. The optical filter 135 is disposed between the two lenses.

The optical filter 135 leads only a fluorescence component to the light receiving sensor 137, and removes an excitation light component (plasmon scattered light δ) in order to detect the fluorescence γ at a high S/N ratio. Examples of the optical filter 135 include an excitation light reflection filter, a short wavelength cut filter, and a bandpass filter. For example, the optical filter 135 is a filter including a multilayer film for reflecting a predetermined light component (light having a predetermined wavelength component) or a colored glass filter for absorbing a predetermined light component.

The light receiving sensor 137 detects the fluorescence γ and the plasmon scattered light δ. The light receiving sensor 137 has such a high sensitivity to be able to detect the weak fluorescence γ from a small amount of detection target substance. For example, the light receiving sensor 137 is a photomultiplier tube (PMT) or an avalanche photodiode (APD) .

The position switching mechanism 132 switches a position of the optical filter 135 between a position on an optical path and a position outside the optical path in the light receiving unit 131. Specifically, the optical filter 135 is disposed on the optical path of the light receiving unit 131 when the light receiving sensor 137 detects the fluorescence γ, and the optical filter 135 is disposed outside the optical path in the light receiving unit 131 when the light receiving sensor 137 detects the plasmon scattered light δ.

The sensor control unit 133 controls detection of an output value of the light receiving sensor 137, management of a sensitivity of the light receiving sensor 137 with the detected output value, change of the sensitivity of the light receiving sensor 137 for obtaining a proper output value, and the like. For example, the sensor control unit 133 is constituted by a known computer or microcomputer including a computing device, a control device, a storage device , an input device, and an output device.

The liquid feeding unit 140 supplies a specimen, a blocking liquid containing a blocking agent, a labeling liquid, a standard liquid, a cleaning liquid, and the like into the channel 41 of the detection chip 10 held by the chip holder 152. The liquid feeding unit 140 includes a liquid chip 141, a syringe pump 142, and a liquid feeding pump drive mechanism 143.

The liquid chip 141 is a container for containing a liquid such as a specimen, a blocking liquid, a labeling liquid, a standard liquid, or a cleaning liquid. As the liquid chip 141, usually, a plurality of containers is disposed according to the kind of liquid, or a chip obtained by integrating a plurality of containers is disposed.

The syringe pump 142 is constituted by a syringe 144 and a plunger 145 capable of being reciprocated in the syringe 144. By the reciprocating motion of the plunger 145, suction of a liquid and ejection thereof are performed quantitatively. When the syringe 144 is replaceable, cleaning of the syringe 144 is not required. Therefore, the replaceable syringe 144 is preferable from a viewpoint of preventing contamination of impurities or the like. When the syringe 144 is not replaceable, by further adding a configuration for cleaning the inside of the syringe 144, the syringe 144 can be used without being replaced.

The liquid feeding pump drive mechanism 143 includes a device for driving the plunger 145 and a device for moving the syringe pump 142. The device for driving the plunger 145 is a device for reciprocating the plunger 145, and for example, includes a stepping motor. A driving device including a stepping motor can manage a liquid feeding amount of the syringe pump 142 or a liquid feeding rate thereof, and is therefore preferable from a viewpoint of managing a residual liquid amount of the detection chip 10. For example, the device for moving the syringe pump 142 freely moves the syringe pump 142 in two directions of an axial direction of the syringe 144 (for example, vertical direction) and a direction crossing the axial direction (for example, horizontal direction). For example, the device for moving the syringe pump 142 is constituted by a robot arm, a 2-axis stage, or a vertically movable turntable.

The liquid feeding unit 140 sucks various liquids from the liquid chip 141, and supplies the liquids into the channel 41 of the detection chip 10. At this time, by moving the plunger 145, a liquid is reciprocated in the channel 41 of the detection chip 10, and the liquid in the channel 41 is stirred. This can make the concentration distribution of the liquid uniform, or can accelerate a reaction in the channel 41 (for example, an antigen-antibody reaction), for example. An inlet of the detection chip 10 is preferably protected by a multilayer film, and the detection chip 10 and the syringe 144 are preferably configured so as to be able to seal the inlet when the syringe 144 penetrates through the multilayer film from a viewpoint of performing such an operation.

The liquid in the channel 41 is sucked by the syringe pump 142 again, and is discharged to the liquid chip 141 or the like. By repeating these operations, a reaction of various liquids, cleaning, and the like can be performed, and a detection target substance labeled with a fluorescent substance can be disposed in a reaction field in the channel 41.

The conveying unit 150 conveys the detection chip 10 to a measurement position or a liquid feeding position, and fixes the detection chip 10. Here, the "measurement position" is a position at which the reflected light detecting unit 120 or the fluorescence detecting unit 130 detects the reflected light β, the fluorescence γ, or the plasmon scattered light δ generated by irradiation to the detection chip 10 with the excitation light α by the excitation light irradiation unit 110. The "liquid feeding position" is a position at which the liquid feeding unit 140 supplies a liquid into the channel 41 of the detection chip 10 or removes the liquid in the channel 41 of the detection chip 10. The conveying unit 150 includes a conveying stage 151 and the chip holder 152. The chip holder 152 is fixed to the conveying stage 151, and holds the detection chip 10 detachably. The chip holder 152 has a shape capable of holding the detection chip 10 and not blocking an optical path of the excitation light α, the reflected light β, the fluorescence γ, or the plasmon scattered light δ. For example, the chip holder 152 includes an opening through which the excitation light α, the reflected light β, the fluorescence γ, and the plasmon scattered light δ pass. The conveying stage 151 moves the chip holder 152 in one direction and in the opposite direction thereto. The conveying stage 151 also has a shape not blocking an optical path of the excitation light α, the reflected light β, the fluorescence γ, or the plasmon scattered light δ. For example, the conveying stage 151 is driven by a stepping motor.

The control unit 160 controls the angle adjusting mechanism 112, the light source control unit 113, the angle adjustment mechanism 122, the sensor control unit 123, the position switching mechanism 132, the sensor control unit 133, the liquid feeding pump drive mechanism 143, and the conveying stage 151. For example, the control unit 160 is constituted by a known computer or microcomputer including a computing device, a control device, a storage device , an input device, and an output device.

### (Detection operation of SPFS device)

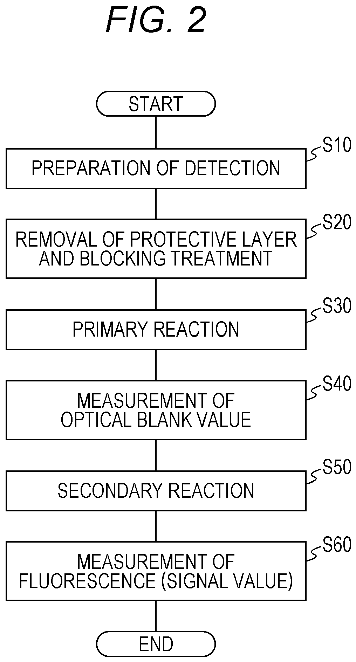

Next, a detection operation of the SPFS device 100 according to the present embodiment (detection method according to an embodiment of the present invention) will be described. For example, the SPFS device 100 can operate according to either of the following two detection operations. Hereinafter, each of the detection operations will be described with reference to the drawings. Fig. 2 is a flowchart illustrating a first operation procedure of the SPFS device 100.

First, detection is prepared (step S10). Specifically, the detection chip 10 is installed in the chip holder 152 of the SPFS device 100.

Subsequently, a protective layer of a capturing body is removed, and an inner surface of the channel 41 is subjected to a blocking treatment (step S20). Specifically, the control unit 160 operates the conveying stage 151, and moves the detection chip 10 to the liquid feeding position. Thereafter, the control unit 160 operates the liquid feeding pump drive mechanism 143, and supplies a blocking liquid of the liquid chip 141 into the channel 41 of the detection chip 10. In the channel 41, the blocking liquid removes a protective layer of a capturing body, moves in the channel 41, and subjects an inner surface of the channel 41 to a blocking treatment. At this time, in order to make the concentration distribution of a blocking agent in the channel 41 more uniform and to accelerate the blocking treatment, the blocking liquid is moved in the channel 41. A method for moving the blocking liquid is not particularly limited. Examples of a method for moving the blocking liquid include repeating suction and ejection alternately with a syringe and irradiation with an ultrasonic wave, a microwave, or the like. A flow rate (moving rate) of the blocking liquid is not particularly limited. When the blocking treatment (step S20) and a primary reaction (step S30) are performed separately, the flow rate of the liquid (blocking liquid) in the channel 41 is preferably 1600 µL/min or more and 3300 µL/min or less. This can make the concentration distribution of a blocking agent in the channel 41 uniform, can accelerate the blocking treatment, and can reduce time required for the blocking treatment. In addition, the concentration of the blocking agent in the blocking liquid is preferably about from 5 to 8% by mass. The blocking agent having a higher concentration is more preferable from a viewpoint of accelerating the blocking treatment. However, the blocking agent having a too high concentration makes the viscosity of the blocking liquid high, and inhibits movement of the blocking liquid. On the other hand, the blocking agent having a too low concentration makes it impossible to perform the blocking treatment properly. Thereafter, the control unit 160 operates the liquid feeding pump drive mechanism 143, and removes the blocking liquid in the channel 41.

The kind of the blocking agent contained in the blocking liquid is not particularly limited as long as the blocking agent can suppress nonspecific bonding of a detection target substance to an inner surface of the channel 41. Examples of the kind of the blocking agent include a high molecular compound such as albumin, casein, skim milk, gelatin, or polyethylene glycol; and a low molecular compound such as a phospholipid, ethylenediamine, or acetonitrile. These blocking agents may be used singly or in combination of two or more kinds thereof.

Following the blocking treatment (step S20), a capturing body is caused to capture a detection target substance (primary reaction; step S30). Here, "following the blocking treatment" means that no other step (for example, a cleaning step or a storage step for a long time) is performed after the blocking treatment. Specifically, the control unit 160 operates the liquid feeding pump drive mechanism 143, and supplies a specimen in the liquid chip 141 into the channel 41. In the channel 41, the detection target substance is captured by the capturing body on the metal film 30 by an antigen-antibody reaction. Thereafter, the control unit 160 operates the liquid feeding pump drive mechanism 143, removes the specimen in the channel 41, and cleans the inside of the channel 41 at least once using a cleaning liquid (for example, a phosphate buffer).

Subsequently, the control unit 160 measures an optical blank value (step S40). Here, the "optical blank value" means a light quantity of background light emitted above the detection chip 10 in measurement of the fluorescence γ described below (step S60). Specifically, the control unit 160 operates the conveying stage 151, and moves the detection chip 10 to a detection position. Thereafter, the control unit 160 operates the excitation light irradiation unit 110 and the fluorescence detecting unit 130, irradiates a back surface of the metal film 30 corresponding to a region to which the capturing body is fixed with the excitation light α through the incident surface 21 so as to cause surface plasmon resonance, and records an output value (optical blank value) of the light receiving sensor 137. At this time, the control unit 160 operates the angle adjusting mechanism 112, and sets an incident angle of the excitation light α to an enhancement angle. In addition, the control unit 160 controls the position switching mechanism 132, and disposes the optical filter 135 in an optical path of the light receiving unit 131. A measured optical blank value is recorded in the control unit 160.

Subsequently, the control unit 160 labels the detection target substance captured on the metal film 30 with a fluorescence substance (secondary reaction; step S50). Specifically, the control unit 160 operates the conveying stage 151, and moves the detection chip 10 to the liquid feeding position. Thereafter, the control unit 160 operates the liquid feeding pump drive mechanism 143, and supplies a liquid labeled with the fluorescence substance and containing the capturing body (labeling liquid) into the channel 41 of the detection chip 10. In the channel 41, the detection target substance captured on the metal film 30 by an antigen-antibody reaction is labeled with the fluorescence substance. Thereafter, the control unit 160 operates the liquid feeding pump drive mechanism 143, removes the labeling liquid in the channel 41, and cleans the inside of the channel 41 using a cleaning liquid.

Subsequently, the control unit 160 measures ae light quantity of the fluorescence γ emitted from the vicinity of a surface of the metal film 30 on a side of the channel 41 (containing unit) to obtain a signal value indicating presence of the detection target substance in the specimen or the amount thereof (step S60). Specifically, the control unit 160 operates the conveying stage 151, and moves the detection chip 10 to a detection position. Thereafter, the control unit 160 operates the excitation light irradiation unit 110 and the fluorescence detecting unit 130, irradiates a back surface of the metal film 30 corresponding to a region to which the capturing body is fixed with the excitation light α through the incident surface 21 from a side of the prism 20 while the detection target substance is bonded to the capturing body and the specimen is not present on the metal film 30, and records an output value of the light receiving sensor 137. At this time, the control unit 160 operates the angle adjusting mechanism 112, and sets an incident angle of the excitation light α to an enhancement angle. In addition, the control unit 160 controls the position switching mechanism 132, and disposes the optical filter 135 in an optical path of the light receiving unit 131. The control unit 160 calculates a signal value indicating presence of the detection target substance in the specimen or the amount thereof by subtracting the optical blank value from a detection value. The signal value is converted into the amount of the detection target substance, the concentration thereof, or the like, if necessary. By the above procedures, presence of the detection target substance in the specimen or the amount thereof can be detected.

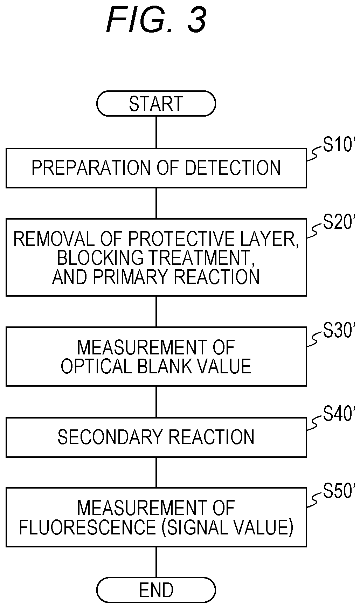

Next, a second detection operation of the SPFS device 100 will be described. Fig. 3 is a flowchart illustrating a second operation procedure of the SPFS device 100.

First, detection is prepared by installing the detection chip 10 in the chip holder 152 of the SPFS device 100 in a similar manner to the above operation procedure (step S10').

Subsequently, a protective layer of a capturing body is removed, and the inside of the channel 41 is subjected to a blocking treatment and a primary reaction simultaneously (step S20'). Specifically, the control unit 160 operates the conveying stage 151, and moves the detection chip 10 to the liquid feeding position. Thereafter, the control unit 160 operates the liquid feeding pump drive mechanism 143, and supplies a mixed liquid of a blocking liquid of the liquid chip 141 and a specimen containing a detection target substance into the channel 41 of the detection chip 10. In the channel 41, the mixed liquid of the blocking liquid and the specimen moves in the channel 41, the protective layer of the capturing body is removed, and an inner surface of the channel 41 is subjected to a blocking treatment. In addition, the detection target substance is captured by the capturing body on the metal film 30 by an antigen-antibody reaction. Also in the present operation procedure, the liquid (mixed liquid of the blocking liquid and the specimen) in the channel 41 is moved in a similar manner to the first operation procedure in order to accelerate the blocking treatment. A flow rate of the liquid (mixed liquid of the blocking liquid and the specimen) in the channel 41 is not particularly limited, but is similar to that in the first operation procedure. On the other hand, for example, the concentration of the blocking agent in the liquid (mixed liquid of the blocking liquid and the specimen) in the channel 41 is preferably about from 2 to 3% by mass. When the blocking treatment and the primary reaction are performed simultaneously, the liquid moving in the channel 41 contains various proteins such as the detection target substance in addition to the blocking agent. Therefore, the viscosity of the liquid is higher than that in a case where the blocking treatment and the primary reaction are performed separately (the concentration of the blocking agent is from 5 to 8% by mass), and it may be difficult to move the liquid properly. Therefore, the concentration of the blocking agent contained in the liquid in the channel 41 is preferably reduced. Thereafter, the control unit 160 operates the liquid feeding pump drive mechanism 143, removes the liquid in the channel 41, and cleans the inside of the channel 41 at least once using a cleaning liquid (for example, a phosphate buffer).

Subsequently, an optical blank value is measured in a similar manner to the above operation procedure (step S30'), the secondary reaction is performed (step S40'), and then a signal value indicating presence of the detection target substance in the specimen or the amount thereof is obtained (step S50'). The control unit 160 calculates a signal value indicating presence of the detection target substance in the specimen or the amount thereof by subtracting the optical blank value from a detection value, and converts the signal value into the amount of the detection target substance, the concentration thereof, or the like, if necessary. By the above procedures, presence of the detection target substance in the specimen or the amount thereof can be detected.

### (Effect)

In the SPFS device 100 according to the present embodiment, by moving the blocking liquid in the channel 41, the concentration of the blocking agent can be uniform. As a result, the blocking treatment is accelerated, and the blocking treatment can be performed in a short time. In addition, in the SPFS device 100 according to the present embodiment, the blocking liquid is moved, and therefore the blocking treatment can be performed properly in a short time regardless of the viscosity of the blocking liquid.

Conventionally, adjustment of the pH of the blocking liquid or the like has been performed from a viewpoint of accelerating the blocking treatment. However, when the pH of the blocking liquid is different from that of the specimen, the detection target substance in the specimen and the blocking agent may be denatured. Therefore, it is necessary to clean the inside of the channel 41 after the blocking treatment in order to remove the blocking liquid from the inside of the channel 41. However, as described above, an effect of the blocking treatment may be reduced by falling off of the blocking agent due to the cleaning liquid. Therefore, it may be impossible to detect the detection target substance with a high accuracy by a conventional detection method. In addition, by performing another step such as a cleaning step, the number of steps before detection of the detection target substance increases, and time required for detection is longer.

In contrast, in the present embodiment, by moving the blocking liquid, the blocking treatment is accelerated. Therefore, it is not necessary to adjust the pH of the blocking liquid. In addition, in the present embodiment, it is not necessary to perform another step such as cleaning the inside of the channel 41 between the blocking treatment and the primary reaction, and therefore there is no risk of falling off of the blocking agent in the other step. Therefore, the SPFS device 100 according to the present embodiment can detect presence of the detection target substance or the amount thereof in a short time with a high accuracy without reducing the effect of the blocking treatment.

In the above embodiment, the detection method and the detection device for bonding excited light to surface plasmon by totally reflecting the excitation light on an interface between the prism 20 and the metal film 30 have been described. However, in the detection method and the detection device according to the present invention, the excitation light may be bonded to the surface plasmon using a diffraction grating. In this case, in the SPFS device 100, it is not always necessary to dispose the metal film 30 on the prism 20, but the SPFS device 100 may include a diffraction grating in place thereof.

In the above embodiment, the detection method and the detection device using SPFS have been described. However, the detection method and the detection device according to the present invention are not limited to the detection method and the detection device using SPFS. For example, the detection method and the detection device according to the present invention may use an SPR method. In this case, the SPFS device 100 detects presence of the detection target substance or the amount thereof by measuring a light quantity of the reflected light β as a signal value without measuring a light quantity of the fluorescence γ. Therefore, measurement of the optical blank value (step S40, step S30') and the secondary reaction (step S50, step S40') are not required.

### Examples

Hereinafter, the present invention will be described in detail with reference to Examples, but is not limited by these Examples.

### [Example 1]

In Example 1, an effect of moving a blocking liquid in a blocking treatment was examined.

### 1. Case where primary reaction is performed following blocking treatment

### (1) Preparation of detection chip

A detection chip in which a reaction field on a metal film (region to which an anti-troponin antibody was fixed as a capturing body) was protected by a protective layer was prepared. A cross-sectional area of a channel on the reaction field was 0.46 mm². The prepared detection chip was installed in a chip holder of an SPFS device.

### (2) Preparation of blocking liquid

A blocking agent (bovine serum albumin (BSA)) was added to an acetate buffer such that a final concentration became 5% by mass to prepare a blocking liquid.

### (3) Blocking treatment

By performing reciprocating liquid feeding of a blocking liquid (100 µL) using a syringe pump in a liquid feeding unit for 35 seconds at a predetermined flow rate (1 to 3300 µL/min) in a channel of the detection chip, a blocking treatment was performed. Subsequently, the blocking liquid in the channel was removed using the syringe pump in the liquid feeding unit.

### (4) Primary reaction

Following the blocking treatment, by performing reciprocating liquid feeding of a diluted liquid of plasma (100 µL) obtained by mixing a phosphate buffer and plasma (specimen) containing troponin (detection target substance) such that a volume ratio thereof was 2 : 1 using the syringe pump in the liquid feeding unit for four minutes at a flow rate of 3300 µL/min in the channel, a primary reaction was performed.

### (5) Confirmation of effect of blocking treatment

After the primary reaction, the metal film was irradiated with light having a wavelength of 635 nm from a side of the prism. Subsequently, reflected light from the metal film was detected, and a reflectance was measured. A blocking ratio was calculated on the basis of a calibration curve between a reflectance and a blocking ratio created in advance and a measured reflectance. When the calibration curve was created, time for the blocking treatment was increased, or the concentration of the blocking agent was increased, and a blocking ratio when no change in the reflectance was observed was assumed to be 100%.

### 2. Case where blocking treatment and primary reaction are performed simultaneously

### (1) Preparation of detection chip

The prepared detection chip was installed in the chip holder of the SPFS device in a similar manner to the above 1. (1) .

### (2) Preparation of mixed liquid of blocking liquid and specimen

A blocking agent (BSA) was added to an acetate buffer to prepare a blocking liquid. The prepared blocking liquid and plasma were mixed such that a volume ratio thereof was 2:1 to prepare a mixed liquid of the blocking liquid and plasma. The final concentration of the blocking agent in the mixed liquid was 2% by mass.

### (3) Blocking treatment and primary reaction

By performing reciprocating liquid feeding of the mixed liquid of the blocking liquid and plasma (100 µL) using the syringe pump in the liquid feeding unit for one minute 28 seconds at a predetermined flow rate (1 to 3300 µL/min) in the channel, the blocking treatment and the primary reaction were performed. Subsequently, the mixed liquid in the channel was removed using the syringe pump in the liquid feeding unit.

### (4) Confirmation of effect of blocking treatment

An inner surface of the channel was subjected to the blocking treatment, and then a blocking ratio was calculated by a similar procedure to the above 1.(5).

### 3. Result

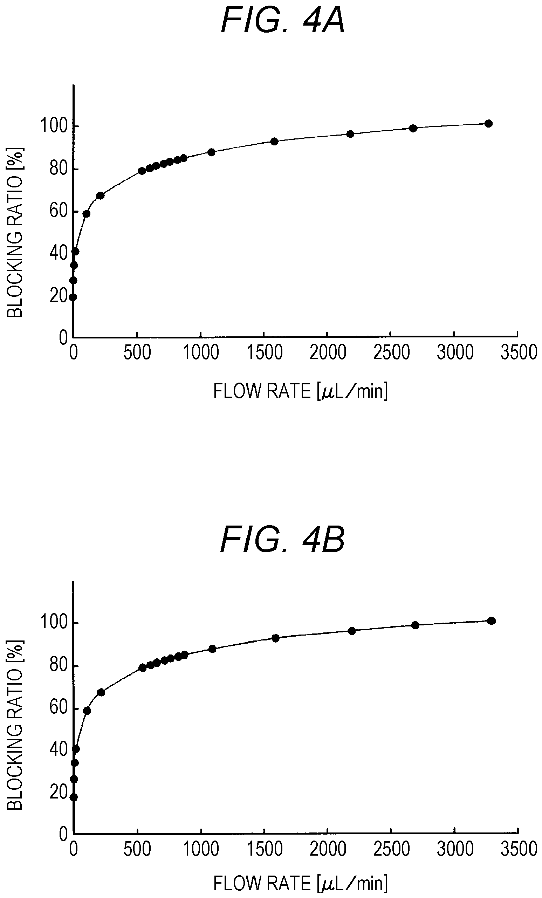

Fig. 4A is a graph indicating a relationship between a flow rate of a blocking liquid and a blocking ratio when the primary reaction is performed following the blocking treatment. Fig. 4B is a graph indicating a relationship between a flow rate of a mixed liquid and a blocking ratio when the blocking treatment and the primary reaction are performed simultaneously. In Figs. 4A and 4B, the horizontal axis represents a flow rate (µL/min) of a liquid (blocking liquid or mixed liquid) in the channel, and the vertical axis represents a blocking ratio (%) .

As illustrated in Figs. 4A and 4B, in both of a case where the primary reaction is performed following the blocking treatment and a case where the blocking treatment and the primary reaction are performed simultaneously, the larger the flow rate of the liquid (blocking liquid or mixed liquid) in the channel was, the higher the blocking ratio was. Particularly, in both of the cases, when the flow rate of the liquid in the channel was 1600 µL/min or more, the blocking ratio was 90% or more. This result indicates that the blocking treatment in the channel can be performed in a short time by moving the liquid in the channel. In addition, the result indicates that the blocking ratio can be 90% or more by setting the flow rate of the liquid in the channel to 1600 µL/min or more and 3300 µL/min or less.

### [Example 2]

In Example 2, a relationship between presence or absence of movement of a blocking liquid and a concentration of a blocking agent, and time required for the blocking treatment when the primary reaction was performed following the blocking treatment was examined.

### (1) Preparation of blocking liquid

A blocking agent (BSA) was added to an acetate buffer such that a final concentration became 0% by mass, 1% by mass, 2% by mass, 5% by mass, or 8% by mass to prepare five kinds of blocking liquids.

### (2) Preparation of detection chip

A plurality of detection chips in each of which a reaction field on a metal film (region to which an anti-troponin antibody was fixed as a capturing body) was protected by a protective layer was manufactured in a similar manner to Example 1. A cross-sectional area of a channel on the reaction field was 0.46 mm². Some of the detection chips were subjected to the blocking treatment by supplying a blocking liquid (100 µL) containing a blocking agent at 1% by mass into the channel immediately after manufacturing and allowing the detection chips to stand for a predetermined time. Thereafter, the blocking liquid was removed from the inside of the channel, and was stored at room temperature and atmospheric pressure for one day or more.

### (3) Blocking treatment

Each of the detection chips which had not been subjected to the blocking treatment immediately after manufacturing was installed in the chip holder of the SPFS device. By performing reciprocating liquid feeding (stirring) of a blocking liquid (100 µL) containing a blocking agent at 0% by mass, 2% by mass, 5% by mass, or 8% by mass using the syringe pump in the liquid feeding unit for a predetermined time at a predetermined flow rate in the channel, the blocking treatment was performed. Subsequently, the blocking liquid in the channel was removed using the syringe pump in the liquid feeding unit.

### (4) Primary reaction

Following the blocking treatment, by performing reciprocating liquid feeding of a diluted liquid of plasma (100 µL) obtained by mixing a phosphate buffer and plasma (specimen) containing troponin (detection target substance) such that a volume ratio thereof was 2:1 for four minutes at a flow rate of 3300 µL/min in the channel, the primary reaction was performed. In addition, each of the detection chips which had been subjected to the blocking treatment immediately after manufacturing was installed in the chip holder of the SPFS device, and then was subjected to the primary reaction by a similar procedure.

### (5) Determination of blocking time

After the primary reaction, a blocking ratio of an inner surface of the channel was calculated by a similar procedure to Example 1. For each condition of each blocking treatment, a lower limit value of treatment time when the blocking ratio was 90% or more (hereinafter, also referred to as "blocking time") was determined.

Table 1 indicates a relationship between a condition of the blocking treatment and the blocking time. Under conditions A and C to E, the blocking treatment was performed immediately before the primary reaction. Under condition B, the blocking treatment was performed immediately after a detection chip was manufactured.

**[Table 1]**

| Condition | Concentration of blocking agent (% by mass) | Treatment method | Flow rate (µL/min) | Blocking time | Category |
|---|---|---|---|---|---|
| A | 0 | Stirred | 3300 | - | Comparative Example |
| B | 1 | Allowed to stand | - | Five hours | Comparative Example |
| C | 2 | Stirred | 3300 | One minute 28 seconds | Example |
| D | 5 | Stirred | 3300 | 35 seconds | Example |
| E | 8 | Stirred | 3300 | 15 seconds | Example |

As indicated in Table 1, the blocking time in a case where the blocking liquid was not moved (condition B) was five hours. On the other hand, the blocking time in a case where the blocking liquid was moved (conditions C to E) was significantly shorter than that in the case where the blocking liquid was not moved (condition B). This result indicates that the blocking time can be largely reduced by moving the blocking liquid. In addition, the result indicates that the higher the concentration of the blocking agent is, the shorter the blocking time can be.

### [Example 3]

In Example 3, a relationship between presence or absence of movement of a blocking liquid and a concentration of a blocking agent, and a signal value when the primary reaction was performed following the blocking treatment was examined. As Comparative Examples, a case where another step was performed between the blocking treatment and the primary reaction was examined.

### (1) Preparation of detection chip

A detection chip which had been subjected to the blocking treatment immediately after manufacturing and a detection chip which had not been subjected to the blocking treatment immediately after manufacturing were prepared by a similar procedure to Example 2 except that the detection chip was allowed to stand for 30 minutes in the blocking treatment immediately after manufacturing. These detection chips were stored at room temperature and atmospheric pressure for one day or more.

### (2) Blocking treatment

Each of the detection chips which had not been subjected to the blocking treatment immediately after manufacturing was installed in the chip holder of the SPFS device. Reciprocating liquid feeding of a blocking liquid (100 µL) containing a blocking agent at 0% by mass, 2% by mass, 5% by mass, or 8% by mass was performed using the syringe pump in the liquid feeding unit for 15 seconds at a flow rate of 3300 µL/min in the channel by a similar procedure to Example 2. Subsequently, the blocking liquid in the channel was removed using the syringe pump in the liquid feeding unit.

Table 2 indicates conditions of the blocking treatment. Under conditions F and H to J, the blocking treatment was performed immediately before the primary reaction. Under condition G, the blocking treatment was performed immediately after a detection chip was manufactured.

**[Table 2]**

| Condition | Concentration of blocking agent (% by mass) | Treatment method | Flow rate (µL/min) | Blocking time | Category |
|---|---|---|---|---|---|
| F | 0 | Stirred | 3300 | 15 seconds | Comparative Example |
| G | 1 | Allowed to stand | - | 30 minutes | Comparative Example |
| H | 2 | Stirred | 3300 | 15 seconds | Example |
| I | 5 | Stirred | 3300 | 15 seconds | Example |
| J | 8 | Stirred | 3300 | 15 seconds | Example |

### (3) Primary reaction

Following the blocking treatment, by performing reciprocating liquid feeding of a diluted liquid of plasma (100 µL) obtained by mixing a phosphate buffer and plasma (specimen) such that a volume ratio thereof was 2:1 using a syringe pump in the liquid feeding unit for four minutes at a flow rate of 3300 µL/min in the channel, the primary reaction was performed. Subsequently, the diluted liquid in the channel was removed using the syringe pump in the liquid feeding unit. In addition, each of the detection chips which had been subjected to the blocking treatment immediately after manufacturing was installed in the chip holder of the SPFS device, and then was subjected to the primary reaction by a similar procedure.

In this experiment, as a specimen, plasma containing a detection target substance (troponin) and plasma containing no troponin were subjected to liquid feeding. Hereinafter, the primary reaction using a specimen containing a detection target substance is also referred to as a "positive reaction", and the primary reaction using a specimen containing no detection target substance is also referred to as a "negative reaction".

### (4) Measurement of optical blank value

After the primary reaction, the inside of the channel was cleaned by introducing a phosphate buffer into the channel. Thereafter, the metal film was irradiated with light having a wavelength of 635 nm from a side of the prism. Light emitted from the vicinity of a surface of the metal film on a side of the channel was detected to obtain an optical blank value.

### (5) Secondary reaction

Thereafter, by performing reciprocating liquid feeding of a solution (100 µL) containing an anti-troponin antibody labeled with a fluorescence dye using the syringe pump in the liquid feeding unit for one minute at a flow rate of 3300 µL/min in the channel, a secondary reaction was performed. In each of a case where the positive reaction was performed and a case where the negative reaction was performed, the secondary reaction was performed. Subsequently, the solution in the channel was removed using the syringe pump in the liquid feeding unit.

### (6) Measurement of signal value indicating amount of detection target substance

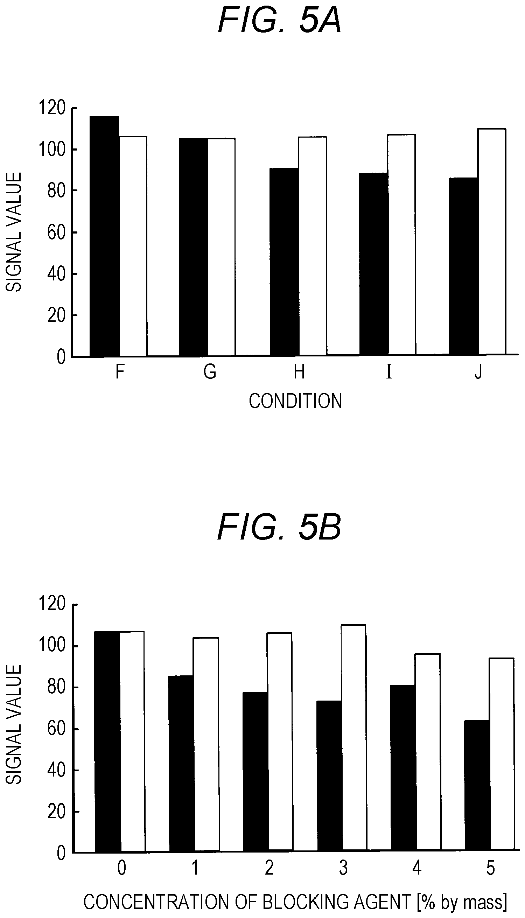

After the inside of the channel was cleaned, fluorescence emitted from the vicinity of a surface of the metal film on a side of the channel was detected in a similar manner to measurement of an optical blank value. A signal value indicating the amount of a detection target substance was obtained by subtracting the optical blank value from a detection value. Fig. 5A illustrates measurement results thereof.

Fig. 5A is a graph indicating a signal value indicating the amount of a detection target substance under conditions F to J. In Fig. 5A, a black bar indicates a result in a case where the negative reaction was performed, and a white bar indicates a result in a case where the positive reaction was performed. The vertical axis represents a signal value (relative value) when a signal value under condition G is assumed to be a reference (100). A signal value in the case where the positive reaction was performed is primarily derived from fluorescence emitted from a fluorescent dye labeling a detection target substance. On the other hand, in the negative reaction, a detection target substance to which a fluorescent dye can be bonded specifically does not exist, and therefore a signal value is derived from a fluorescent dye or other autofluorescence non-specifically bonded to the inside of the channel. That is, the signal value in the case where the positive reaction was performed indicates the amount of a detection target substance, and the signal value in the case where the negative reaction was performed indicates the amount of noise.

In Fig. 5A, when results of the negative reactions under conditions G to J are compared with one another, a signal value (noise) of a detection chip which has been subjected to the blocking treatment immediately after manufacturing (condition G) is higher than signal values (noise) of detection chips which have been subjected to the blocking treatment immediately before the primary reaction (conditions H to J) . This result indicates that an effect of the blocking treatment is reduced by storing a detection chip for a long time after the detection chip is subjected to the blocking treatment. This indicates that it is preferable to perform the blocking treatment immediately before the primary reaction without performing another step between the blocking treatment and the primary reaction from a viewpoint of improving an SN ratio.

In Fig. 5A, when results of the negative reactions under conditions F and H to J are compared with one another, the higher the concentration of the blocking agent is, the lower the signal value (noise) is. Particularly, when results of the negative reactions under conditions F and J are compared with each other, the signal value (noise) is reduced about by 30%. When results of the negative reactions under conditions G and J are compared with each other, by moving the blocking liquid, the signal value (noise) is reduced about by 20%. These results indicate that movement of the blocking liquid and a higher concentration of the blocking agent make an effect of the blocking treatment higher, and can suppress nonspecific bonding of a detection target substance to an inner surface of the channel.

On the other hand, when results of the positive reactions under conditions F to J are compared with one another, the signal value is not reduced even when the concentration of the blocking agent is increased. This indicates that the concentration of the blocking agent has no influence on the amount of a detection target substance captured by a capturing body when the primary reaction is performed following the blocking treatment.

The above results indicate that time required for detecting a detection target substance (including time required for the blocking treatment) can be reduced, the SN ratio can be improved, and the amount of the detection target substance can be detected with a high accuracy by moving the blocking liquid and increasing the concentration of the blocking agent.

### [Example 4]

In Example 4, a relationship between a concentration of the blocking agent and a signal value when the blocking treatment and the primary reaction were performed simultaneously was examined.

### (1) Preparation of detection chip

A detection chip in which a reaction field on a metal film (region to which an anti-troponin antibody was fixed as a capturing body) was protected by a protective layer was installed in the chip holder of the SPFS device in a similar manner to Example 1. A cross-sectional area of a channel on the reaction field was 0.46 mm².

### (2) Preparation of mixed liquid of blocking liquid and specimen

A blocking agent (BSA) was added to an acetate buffer to prepare a blocking liquid. The prepared blocking liquid and plasma (specimen) were mixed such that a volume ratio thereof was 2:1 to prepare six kinds of mixed liquids of the blocking liquid and plasma. The final concentrations of the blocking agent in the mixed liquids were 0% by mass, 1% by mass, 2% by mass, 3% by mass, 4% by mass, and 5% by mass. At this time, by using plasma containing a detection target substance (troponin) and plasma containing no troponin as a specimen, 12 kinds of mixed liquids were prepared.

### (3) Blocking treatment and primary reaction

Reciprocating liquid feeding of a mixed liquid (100 µL) was performed using the syringe pump in the liquid feeding unit for four minutes at a flow rate of 3300 µL/min in the channel. Subsequently, the mixed liquid in the channel was removed using the syringe pump in the liquid feeding unit.

### (4) Measurement of signal value indicating amount of detection target substance

Measurement of an optical blank value and the secondary reaction were performed by a similar procedure to Example 2 to obtain a signal value indicating the amount of a detection target substance. Fig. 5B illustrates measurement results thereof.

Also in Fig. 5B, a black bar indicates a result in a case where the negative reaction was performed, and a white bar indicates a result in a case where the positive reaction was performed. The horizontal axis represents a concentration (% by mass) of the blocking agent. The vertical axis represents a signal value (relative value) when a signal at a concentration of the blocking agent of 0% by mass (without containing the blocking agent) is assumed to be a reference (100).

When results of the negative reactions are compared with one another, the signal value (noise) at a concentration of the blocking agent of 0% by mass is the highest, and there is a tendency that the higher the concentration of the blocking agent is, the lower the signal value (noise) is. This result indicates that a higher concentration of the blocking agent makes an effect of the blocking treatment higher, and can suppress nonspecific bonding in the channel.

On the other hand, when results of the positive reactions are compared with one another, in a case where the concentration of the blocking agent is higher than 3% by mass, the higher the concentration of the blocking agent is, the lower the signal value is. This result suggests that the concentration of the blocking agent higher than 3% by mass reduces the amount of a detection target substance bonded to a capturing body. It is considered that the primary reaction tends to be inhibited because the concentration of the blocking agent higher than 3% by mass increases the viscosity of the mixed liquid and suppresses movement of a detection target substance.

The above results indicate that the concentration of the blocking agent in the mixed liquid is preferably 3% by mass or less in order to prevent the primary reaction from being inhibited when the blocking treatment and the primary reaction are performed simultaneously.

### Industrial applicability

The method and device for detecting a detection target substance using the reaction method according to the present invention can detect the detection target substance with a high reliability, and therefore are useful for examination of a disease, for example.

### Reference Signs List

- 10: detection chip
- 20: prism
- 21: incident surface
- 22: film forming surface
- 23: light emitting surface
- 30: metal film
- 40: channel lid
- 41: channel
- 100: SPFS device
- 110: excitation light irradiation unit
- 111: light source unit
- 112: angle adjusting mechanism
- 113: light source control unit
- 120: reflected light detecting unit
- 121: light receiving sensor
- 122: angle adjusting mechanism
- 123: sensor control unit
- 130: fluorescence detecting unit
- 131: light receiving unit
- 132: position switching mechanism
- 133: sensor control unit
- 134: first lens
- 135: optical filter
- 136: second lens
- 137: light receiving sensor
- 140: liquid feeding unit
- 141: liquid chip
- 142: syringe pump
- 143: liquid feeding pump drive mechanism
- 144: syringe
- 145: plunger
- 150: conveying unit
- 151: conveying stage
- 152: chip holder
- 160: control unit
- α: excitation light
- β: reflected light
- γ: fluorescence
- δ: plasmon scattered light

## Claims

1. A detection method for detecting the presence or the amount of a detection target substance in a specimen by utilizing surface plasmon resonance using a detection chip (10) including a channel (41) for containing a liquid, the channel including a metal film (30) with a reaction field which is exposed to the channel (41) and a capturing body fixed to the metal film, wherein the metal film is capable of causing surface plasmon resonance, the detection method comprising:
a step of controlling a liquid feeding unit to subject the inside of the channel (41) to a blocking treatment (S20) by moving a blocking liquid containing a blocking agent in the channel (41) to stir the blocking liquid within the channel, and wherein a flow rate of the blocking liquid in the channel (41) is 1600 µL/min or more and 3300 µL/min or less and the concentration of the blocking agent in the blocking liquid is from 5% to 8% by mass;
a step of controlling the liquid feeding unit to supply the specimen into the channel (41), causing the capturing body to capture the detection target substance;
and a step of detecting (S60, S50') a light quantity of fluorescence (γ) emitted from the vicinity of a surface of the metal film (30) located on a side of the channel (41) or a light quantity of light reflected by the metal film (30) located on a side of the channel by irradiating the metal film with light such that surface plasmon resonance occurs on the metal film while the detection target substance is bonded to the capturing body, wherein
the step of supplying the specimen, causing the capturing body to capture the detection target substance (S30), is performed following the step of performing the blocking treatment (S20), with no step of cleaning the channel being performed between the step performing the blocking treatment and the step of causing the capturing body to capture the detection target substance.

2. The detection method according to claim 1, wherein
a cross-sectional area of the channel (41) in a cross section perpendicular to a flowing direction of the liquid on the reaction field is 0.01 mm² or more and 100 mm² or less.

3. A detection device (100) for detecting the presence or the amount of a detection target substance in a specimen by utilizing surface plasmon resonance using a detection chip (10), the detection device comprising:
a detection chip (10) including a channel (41) for containing a liquid, the channel further including a metal film with a reaction field which is exposed to the channel (41) and a capturing body fixed to the metal film;
a holder (152) holding the detection chip (10);
a liquid feeding unit (140) for supplying a liquid into the channel (41) while the detection chip (10) is held by the holder (152);
a light irradiation unit (110) for irradiating the metal film (30) of the detection chip (10), held by the holder (152) with light, such that surface plasmon resonance occurs on the metal film (30);
a control unit (160); and
a light detection unit for detecting a light quantity of fluorescence (γ) emitted from the vicinity of a surface of the metal film (30) located on a side of the channel (41) or a light quantity of light reflected by the metal film (30) located on a side of the channel, when the light irradiation unit irradiates the metal film (30) with light; a supply of blocking liquid;
wherein the control unit (160) is configured to control the liquid feeding unit (140) to perform:
a step of subjecting the inside of the channel (41) to a blocking treatment by supplying the blocking liquid containing a blocking agent into the channel (41) and moving the blocking liquid in the channel (41) to stir the blocking liquid within the channel, and wherein a flow rate of the blocking liquid moved by the liquid feeding unit (140) in the channel is 1600 µL/min or more and 3300 µL/min or less and a concentration of the blocking agent in the blocking liquid is from 5% to 8%, by mass; and
a step of supplying the specimen into the channel (41), causing the capturing body to capture the detection target substance, and
wherein the control unit is further configured to control the liquid feeding unit (140) to perform the step of supplying the specimen, causing the capturing body to capture the detection target substance following the step of performing the blocking treatment, with no step of cleaning the channel being performed between the step of performing the blocking treatment and the step of causing the capturing body to capture the detection target substance.

4. The detection device (100) according to claim 3, wherein a cross-sectional area of the channel (41) in a cross section perpendicular to a flowing direction of the liquid on the reaction field is 0.01 mm² or more and 100 mm² or less.

## Patentansprüche

1. Nachweisverfahren zum Nachweis des Vorhandenseins oder der Menge einer Nachweiszielsubstanz in einer Probe durch Verwendung von Oberflächenplasmonenresonanz unter Verwendung eines Detektorchips (10), der einen Kanal (41) zur Aufnahme einer Flüssigkeit umfasst,
wobei der Kanal einen Metallfilm (30) mit einem Reaktionsfeld, das dem Kanal (41) ausgesetzt ist, und einen auf dem Metallfilm befestigten Aufnahmekörper umfasst,
wobei der Metallfilm in der Lage ist, Oberflächenplasmonenresonanz zu verursachen,
wobei das Nachweisverfahren umfassend aufweist:
einen Schritt zum Steuern einer Flüssigkeitszuführeinheit, um das Innere des Kanals (41) einer Blockierbehandlung (S20) zu unterziehen, indem eine Blockierflüssigkeit, die ein Blockiermittel enthält, in dem Kanal (41) bewegt wird, um die Blockierflüssigkeit innerhalb des Kanals zu rühren, und wobei eine Strömungsrate der Blockierflüssigkeit in dem Kanal (41) 1600 µL/min oder mehr und 3300 µL/min oder weniger beträgt und die Konzentration des Blockiermittels in der Blockierflüssigkeit 5 bis 8 Massenprozent beträgt;
einen Schritt zum Steuern der Flüssigkeitszuführeinheit, um die Probe in den Kanal (41) zuzuführen, wodurch der Aufnahmekörper veranlasst wird, die Nachweiszielsubstanz einzufangen;
einen Schritt des Nachweises (S60, S50') einer Lichtmenge an Fluoreszenz (γ), die aus der Nähe einer Oberfläche des Metallfilms (30), der sich auf einer Seite des Kanals (41) befindet, emittiert wird, oder einer Lichtmenge an Licht, die von dem Metallfilm (30), der sich auf einer Seite des Kanals befindet, reflektiert wird, durch Bestrahlen des Metallfilms mit Licht, so dass Oberflächenplasmonenresonanz auf dem Metallfilm auftritt, während die Nachweiszielsubstanz an den Aufnahmekörper gebunden ist,
wobei der Schritt des Zuführens der Probe, das Veranlassen des Eufnahmekörpers, die Nachweiszielsubstanz einzufangen (S30), nach dem Schritt des Ausführens der Blockierungsbehandlung (S20) durchgeführt wird, wobei zwischen dem Schritt des Ausführens der Blockierungsbehandlung und dem Schritt des Veranlassens des Aufnahmekörpers, die Nachweiszielsubstanz einzufangen, kein Schritt des Reinigens des Kanals durchgeführt wird.

2. Nachweisverfahren gemäß Anspruch 1, wobei eine Querschnittsfläche des Kanals (41) in einem Querschnitt senkrecht zu einer Fließrichtung der Flüssigkeit auf dem Reaktionsfeld 0,01 mm² oder mehr und 100 mm² oder weniger beträgt.

3. Nachweisvorrichtung (100) zum Nachweisen des Vorhandenseins oder der Menge einer Nachweiszielsubstanz in einer Probe unter Verwendung von Oberflächenplasmonenresonanz unter Verwendung eines Nachweischips (10), wobei die Nachweisvorrichtung aufweist:
einen Detektorchip (10), der einen Kanal (41) zum Aufnehmen einer Flüssigkeit Reaktionsfeld, wobei der Kanal ferner einen Metallfilm mit einem Reaktionsfeld, das dem Kanal (41) ausgesetzt ist, und einen an dem Metallfilm befestigten Aufnahmekörper umfasst;
einen Halter (152), der den Detektorchip (10) hält;
eine Flüssigkeitszuführeinheit (140) zum Zuführen einer Flüssigkeit in den Kanal (41), während der Detektorchip (10) von dem Halter (152) gehalten wird;
eine Lichtbestrahlungseinheit (110) zum Bestrahlen des Metallfilms (30) des von dem Halter (152) gehaltenen Detektorchips mit Licht, so dass auf dem Metallfilm (30) eine Oberflächenplasmonenresonanz auftritt;
eine Steuereinheit (160); und
eine Lichterfassungseinheit zum Erfassen einer Lichtmenge an Fluoreszenz (γ), die aus der Nähe einer Oberfläche des Metallfilms (30), der sich auf einer Seite des Kanals (41) befindet, emittiert wird, oder einer Lichtmenge an Licht, die von dem Metallfilm (30), der sich auf einer Seite des Kanals befindet, reflektiert wird, wenn die Lichtbestrahlungseinheit den Metallfilm (30) mit Licht bestrahlt;
eine Zufuhr von Sperrflüssigkeit;
wobei die Steuereinheit (160) konfiguriert ist, um die Flüssigkeitszuführeinheit (140) zum Ausführen zu steuern:
einen Schritt des Unterwerfens des Inneren des Kanals (41) einer Blockierungsbehandlung durch Zuführen der Blockierungsflüssigkeit, die ein Blockierungsmittel enthält, in den Kanal (41) und Bewegen der Blockierungsflüssigkeit in dem Kanal (41), um die Blockierungsflüssigkeit in dem Kanal zu rühren, und wobei eine Strömungsrate der von der Flüssigkeitszuführeinheit (140) in dem Kanal bewegten Blockierungsflüssigkeit 1600 µL/min oder mehr und 3300 µL/min oder weniger beträgt und eine Konzentration des Sperrmittels in der Sperrflüssigkeit 5 bis 8 Masseprozent; und
einen Schritt des Zuführens der Probe in den Kanal (41), der bewirkt, dass der Aufnahmekörper die Nachweiszielsubstanz einfängt, und
wobei die Steuereinheit ferner konfiguriert ist, um die Flüssigkeitszuführeinheit (140) zu steuern, um den Schritt des Zuführens der Probe durchzuführen, wodurch der Aufnahmekörper veranlasst wird, die Nachweiszielsubstanz nach dem Schritt des Ausführens der Blockierungsbehandlung einzufangen, wobei zwischen dem Schritt des Ausführens der Blockierungsbehandlung und dem Schritt des Veranlassens des Aufnahmekörpers, die Nachweiszielsubstanz einzufangen, kein Schritt des Reinigens des Kanals durchgeführt wird.

4. Nachweisvorrichtung (100) zum Nachweis gemäß Anspruch 3, wobei eine Querschnittsfläche des Kanals (41) in einem Querschnitt senkrecht zu einer Fließrichtung der Flüssigkeit auf dem Reaktionsfeld 0,01 mm² oder mehr und 100 mm² oder weniger beträgt.

## Revendications

1. Procédé de détection pour détecter la présence ou la quantité d'une substance cible de détection dans un échantillon en utilisant la résonance plasmonique de surface en utilisant une puce de détection (10) comprenant un canal (41) pour contenir un liquide, le canal comprenant un film (30) métallique avec un champ de réaction qui est exposé au canal (41) et un corps de capture fixé au film métallique, dans lequel le film métallique est capable de provoquer une résonance plasmonique de surface, le procédé de détection comprenant :
une étape de commande d'une unité d'alimentation en liquide à soumettre l'intérieur du canal (41) vers un traitement de blocage (S20) en déplaçant un liquide de blocage contenant un agent de blocage dans le canal (41) pour agiter le liquide de blocage à l'intérieur du canal, et dans lequel un débit du liquide de blocage dans le canal (41) est de 1600 µL/min ou plus et de 3300 µL/min ou moins et la concentration de l'agent de blocage dans le liquide de blocage est de 5% à 8% en masse ;
une étape de commande de l'unité d'alimentation en liquide pour fournir l'échantillon dans le canal (41), obligeant le corps de capture à capturer la substance cible de détection, et
une étape de détection (S60, S50') d'une quantité lumineuse de fluorescence (y) émise par le voisinage d'une surface du film métallique (30) située sur un côté du canal (41) ou d'une quantité de lumière lumineuse réfléchie par le film métallique (30) située sur un côté du canal en irradiant le film métallique avec de la lumière de telle sorte que la résonance plasmonique de surface se produit sur le film métallique tandis que la substance cible de détection est liée au corps de capture,
dans lequel
l'étape de fourniture de l'échantillon, amenant le corps de capture à capturer la substance cible de détection (S30) est exécutée après l'étape de réalisation du traitement de blocage (S20), sans qu'aucune étape de nettoyage du canal ne soit effectuée entre l'étape d'exécution du traitement de blocage et l'étape consistant à amener le corps de capture à capturer la substance cible de détection.

2. Procédé de détection selon la revendication 1, dans lequel
une surface de section transversale du canal (41) dans une section transversale perpendiculaire à une direction d'écoulement du liquide sur le champ de réaction est de 0,01 mm² ou plus, et 100 mm² ou moins.

3. Dispositif de détection (100) pour détecter la présence ou la quantité d'une substance cible de détection dans un échantillon en utilisant la résonance plasmonique de surface à l'aide d'une puce de détection (10), le dispositif de détection comprenant :
une puce de détection (10) comprenant un canal (41) pour contenir un liquide, le canal comprenant en outre un film métallique avec un champ de réaction qui est exposé au canal (41) et un corps de capture fixé au film métallique ;
un support (152) pour maintenir la puce de détection (10) ;
une unité d'alimentation en liquide (140) pour fournir un liquide dans le canal (41) tandis que la puce de détection (10) est maintenue par le support (152) ;
une unité d'irradiation lumineuse (110) pour irradier le film métallique (30) de la puce de détection (10), maintenu par le support (152) avec de la lumière, de telle sorte qu'une résonance plasmonique de surface se produit sur le film métallique (30);
une unité de commande (160) ; et
une unité de détection de lumière pour détecter une quantité de lumière de fluorescence (y) émise à proximité d'une surface du film métallique (30) située sur un côté du canal (41) ou une quantité de lumière lumineuse réfléchie par le film métallique (30) situé sur un côté du canal, lorsque l'unité d'irradiation lumineuse irradie le film métallique (30) avec de la lumière ;
une réserve de liquide de blocage ;
dans lequel
l'unité de commande (160) est configurée pour commander l'unité d'alimentation en liquide (140) pour effectuer :
une étape de soumission de l'intérieur du canal (41) à un traitement de blocage en fournissant le liquide de blocage contenant un agent de blocage dans le canal (41) et déplacer le liquide de blocage dans le canal (41) pour agiter le liquide de blocage à l'intérieur du canal, et dans lequel un débit du liquide de blocage déplacé par l'unité d'alimentation en liquide (140) dans le canal est de 1600 µL/min ou plus et 3300 µL/min ou moins et une concentration de l'agent bloquant dans le liquide bloquant est de 5% à 8%, en masse ; et
une étape de fourniture de l'échantillon dans le canal (41), amenant le corps de capture à capturer la substance cible de détection, et
dans lequel l'unité de commande est en outre configurée pour commander l'unité d'alimentation en liquide (140) pour effectuer l'étape de fourniture de l'échantillon, amenant le corps de capture à capturer la substance cible de détection après l'étape de réalisation du traitement de blocage, sans qu'aucune étape de nettoyage du canal ne soit effectuée entre l'étape de réalisation du traitement de blocage et l'étape consistant à amener le corps de capture à capturer la substance cible de détection.

4. Dispositif de détection (100) selon la revendication 3, dans lequel une surface de section transversale du canal (41) dans une section transversale perpendiculaire à une direction d'écoulement du liquide sur le champ de réaction est de 0,01 mm² ou plus, et 100 mm² ou moins.
